# EUROPEAN PATENT SPECIFICATION

(11) **EP 1 091 735 B1**
(45) Date of publication and mention of the grant of the patent: **14.11.2007**
(21) Application number: 99938697.2
(22) Date of filing: 21.06.1999
(51) Int. Cl.: A61K 38/18, A61K 35/16, A61K 38/48, A61K 31/375, A61K 31/355, A61K 31/05, A61P 17/02

(54) **IMPROVED ENRICHED PLATELET WOUND HEALANT**
VERBESSERTES ANGEREICHERTES BLUTPLÄTTCHEN WUNDHEILMITTEL
CICATRISANT POUR BLESSURES AMELIORE A L'AIDE DE PLAQUETTES ENRICHIES

(30) Priority: 22.06.1998 US 90167 P; 26.08.1998 US 97897 P; 13.02.1999 WO PCT/US99/02981
(43) Date of publication of application: 18.04.2001
(73) Proprietor: Cytomedix, Inc., Deerfield, Illinois 60015 (US)
(72) Inventor: WORDEN, Charles, E., Little Rock, AR 72211 (US)
(74) Representative: Abnett, Richard Charles
(86) International application number: PCT/US1999/013958
(87) International publication number: WO 1999/066923

(56) References cited:
- EP-A- 0 493 985
- WO-A-86/03122
- WO-A-98/55140
- WO-A-99/66797
- FR-A- 2 667 789
- US-A- 4 427 651
- US-A- 5 011 695
- US-A- 5 165 938
- US-A- 5 510 102
- US-A- 5 585 007
- US-A- 5 607 694
- US-A- 5 674 912
- US-A- 5 733 545
- DATABASE HCAPLUS ON STN, (Columbus, Ohio, USA), 103:59296, MANN, Wound Healing Compositions.
- DATABASE HCAPLUS ON STN, (Columbus, Ohio, USA), 118:225439, SAIKA S., "Effect of L-ascorbic Acid 2-Phosphate on Corneal Wound Healing", WAKAYAMA IGAKU, 1992.
- BIOSIS ON STN, (Columbus, Ohio, USA), 1993:356018, CLEWELL K., "Topically Applied Ascorbic Acid Enhances Wound Healing and Production of Connective Tissue Proteins"; & JOURNAL OF INVESTIGATIVE DERMATOLOGY, 1993.
- DATABASE HCAPLUS ON STN, (Columbus, Ohio, USA), 126:135685, MacPHEE et al., (AMERICAN NATIONAL RED CROSS, USA), Supplemented and Unsupplemented Tissue Sealants, Methods of Their Production and use See Abstract.

## Description

### BACKGROUND OF THE INVENTION

The invention disclosed herein generally relates to a composition of matter used in the treatment of wounds and a method of making same.

There have been many different substances and methods developed in the past for treating wounds, depending upon the type and location and severity of the wound. A wound is generally defined as an injury to an area of the body of a human or animal. Although injury to the surface of the skin is the most well known type of wound, the surfaces of internal organs may also be wounded, such as during surgery, rupture of the spleen or liver, or resulting from traumatic blows to the body surface in the vicinity of an internal organ.

Medical practice characterizes wounds as chronic or acute, according to the persistency and severity of the wound. A chronic wound is one that is prolonged or lingering, rather than promptly healed. An acute wound is one that occurs relatively quickly, and heals relatively quickly as well. Tissue wounds may have a wide spectrum of manifestations, as small as merely an abnormal microscopic tear or fissure in tissue (or a surface thereof), or as large as the abrasion or ablation of the skin covering a substantial portion of the body, such as in a bum victim. Acute wounds covering a large or movable surface are usually the most difficult to guard from infection, and to heal.

The invention described herein is primarily related to substances topically applied to the exterior surface ofchronic wounds, although the invention described herein also has some applications for facilitating the healing of other wounds such as acute wounds. The composition of matter described herein is especially suited to topical application to bum wounds and chronic lesions, such as ulcers on the feet of diabetics. However, the compositions of matter and the methods described herein are not limited solely to that topical application.

Wound healing is affected by the presence of various substances found in the blood and bodily fluids. The blood is the primary medium for delivering healing agents to the wound site, and for transporting foreign or harmful substances away from the wound. Whole blood is primarily comprised of three main types of cells suspended in a protein rich solution known as plasma.

The three main cell types of whole blood are erythrocytes (a.k.a. red blood cells), leukocytes (a.k.a. white blood cells) and thrombocytes (a.k.a. platelets). The red blood cells are the iron-containing cells that facilitate the transport and transfer of oxygen to body tissue, and the removal of carbon dioxide. The white blood cells perform functions such as phagocytosis of foreign bodies and production of antibodies, primarily responsible for fighting infection and foreign substances within the blood or wound site. Platelets perform many functions such as plugging leaks in blood vessels and helping begin the process leading to the formation of a blood clot; platelets contain substances known as growth factors that facilitate the formation of new tissue.

Although there are several methods for separating whole blood into its various components, one of the most convenient and expeditious methods is accomplished by differentially centrifuging blood or some of its components (i.e., apheresis). In this way, the red and white blood cells and plasma may be separated out and returned to the donor's or patient's body, leaving the sequestered platelets in essentially concentrated form for use in wound healing techniques. From blood extracted from a patient, the platelets may thus be obtained and activated for use on the same patient; methods of using a patient's own blood are called "autologous" or "autogenic" donor methods. Methods using blood donated by one or more third parties for use by a patient are called "homologous" or "heterologous" donor methods, or collectively called "allogenic" methods.

One of the proteins suspended in plasma is fibrinogen, which reacts with substances released into (or attracted by) wound sites to produce sticky strands of fibrin. Such reactions result in the cross linking of the strands to form a mesh that holds and supports the deposit or growth of other tissue materials at the wound site.

The wound healing process is generally considered to occur in several stages, generally known as the healing cascade. After tissue injury, platelets are among the first cells to appear in the vicinity of the wound. Activation of a platelet by an agonist such as thrombin, or other agonists such as those listed elsewhere herein, leads to the release of granule material from within the platelet. Such granulation activation results in the release of proteins known as growth factors, primarily concentrated in the alpha granules of platelets. These released growth factors stimulate the formation of new tissue; when applied to wounds, growth factors have been known to increase the rate of collagen laydown, vascular ingrowth, fibroblast proliferation and overall healing. The release of a protein known as platelet-derived growth factor (PDGF) is a chemotactic for monocytes, neutrophils and fibroblasts into the wound, to begin the inflammatory stage of the healing process. During this time, monocytes secrete PDGF and another platelet protein known as transforming growth factor-β1, which recruits and activates fibroblasts, a precursor to fibrinogen, to begin the repair stage of the healing process. Subsequently, wound healing continues through the process of collagen remodeling within the wound.

The presence of growth factors promotes wound healing. The invention described herein increases the amount of growth factors in the wound, and thereby facilitates the promotion of the healing rate. This may be especially important in "wounded" patients, especially those with chronic wounds who may lack sufficient circulation to facilitate the healing cascade. The invention described herein also facilitates the covering of the wound area with a substance that prevents or helps to reduce infection caused by most bacteria; and to the extent that the wound treatment material is made from autologous blood or similar biological materials, the invention described herein reduces the risks associated with the use of treatment materials made from biological materials obtained from one or more third parties. An autologous product avoids some of the common problems associated with the use of biological materials from third parties, such as (for example) screening to assure that the donor was biologically or immunologically compatible with the patient, and otherwise free of hepatitis, HIV and the like.

Base upon the foregoing general scientific principles, already known in the field are wound sealants made from biological materials obtained primarily from tissue other than blood platelets. For example, wound sealants include "fibrin glue," which often is essentially a mixture of co-coagulants (thrombin and calcium), concentrated fibrinogen and other coagulation proteins. In most applications, the primary roles of fibrin glue are to seal wound surfaces to prevent loss of blood and other body fluids after surgery, and to provide adhesion between adjacent tissue surfaces. These products form a hard, cast-like covering over the area to be sealed, and tend to be non-yielding to limb movement.

The production offibrin glue often requires obtaining fibrinogen from blood through a process known as cryoprecipitation, including both freeze-thaw cycles and relatively lengthy centrifugation of plasma in controlled environments, to concentrate the fibrinogen in large enough amounts required for use; the precipitant thus obtained is frozen to -20° to -30° centigrade before storage. These requirements make such materials unsuitable for application during the course of surgery, especially emergency surgery without an hour or more lead time; moreover, to the extent this process depends upon the use of autologous biological materials, using this process shortly before or during surgery may result in the loss of crucial bodily fluids during a time when the patient's body is badly in need of such fluids. By contrast, substantially larger amounts of concentrated platelets can be more conveniently obtained within a matter of minutes from more recent methods of differential blood centrifugation not requiring freezing, without significant loss of bodily fluids.

To date, there has been much research concerning fibrin glue. This is considered to be a separate field from the present invention, primarily because fibrin glues typically contain cryoprecipitated proteins without platelets. The use of fibrin glue is discussed extensively in the scientific literature; for example, see the references cited in U.S. patent number 5,585,007 issued to Antanavich et al on December 17, 1996.

One method of differential centrifugation essentially allows separating the patient's own blood into at least three different components: packed erythrocytes (red blood cells), plasma and platelet concentrate. Platelet concentrate can be combined with a solution of either sodium or calcium mixed with thrombin ("calcified thrombin"), often to form a gelatinous composition of activated platelets that, when made with the necessary viscosity, can be utilized as a wound sealant. Such sealants typically set up into a hard mass covering the application site, thereby sealing the site. The initially sticky, gelatinous state usually hardens to serve the functions of (1) stopping the loss of blood and other bodily fluids, because it effectively acts as a patch; (2) sealing wounds against external contaminants; and (3) preventing traditional problems associated with the mere stitching of wounds.

Wound healing compositions including platelets have advantages over materials without platelets. One reason is that natural wound healing agents are released by the platelets. Further, the concentration of platelets likewise allows for a concentrated amount of wound healing factors. Additionally, to the extent that the wound healing composition is made from the biological materials of the patient, the risks associated with heterologous donors (such as disease, immunologic reactions, or the like) are eliminated.

The work surrounding the field of autologous platelet gel to date has focused on perioperative blood treatment (hemostatic effect) - preventing loss of blood during or immediately following surgery. Normally, when a patient is on the operating table, the patient will lose large amounts of blood and other bodily fluids, depending upon the type of surgery involved. To counter this blood loss, the traditional approach is to infuse the patient with blood, which is usually donated from one or more third parties (or sometimes donated by the patient in anticipation of surgical needs). There exists many different types of methods for collecting blood that are normally used in this type situation.

Because there is obviously an increased risk of disease, immunologic reaction, or other complications associated with procedures including heterologous blood donation, recent efforts have been made to use blood contemporaneously obtained from the patient during surgery. This blood can be fractionated and/or filtered, and subsequently re-infused into the patient, saving much time, expense, bodily fluids and avoiding normal risks discussed above.

It is from the perioperative blood treatment arena that the uses for autologous platelet gel were focused. The use of platelet gel on open wounds resulting from surgery have recently met great success. This particular use allows the patient to keep his/her own blood and also reduce costs.

The following patents are arguably related to the invention disclosed herein:

| **Patent Number** | **Inventor** | **Date** |
|---|---|---|
| 5,733,545 | Hood | March 31,1998 |
| 5,585,007 | Antanavich, et al. | December 17, 1996 |
| 5,165,938 | Knighton | November 24, 1992 |
| 5,674,912 | Martin | October 7, 1997 |

However, the inventions disclosed therein are patentably distinct from the invention disclosed herein.

The Hood patent claims a plasma-buffy coat concentrate comprising plasma, platelets (at a concentration of at least 10⁹ cells/ml), fibrinogen (at a concentration of at least 5 mg/ml), and white blood cells (at a concentration of at least 3 times 10⁷ cells/ml). The Hood invention achieves hemoconcentration by removal of water from plasma. The Hood invention also fails to recognize the benefits of increased levels of vitamins, antibiotics and other substances. For instance, higher amounts of vitamin C is believed to prolong the viscosity and longevity of a gelatinous composition of fibrinous matter derived substantially from platelets. As another example, the Hood invention fails to recognize the benefits of including retinoids, such as vitamin A (retinol) and/or vitamin E. For instance, patients undergoing treatment including steroids often have immune systems that are suppressed, or otherwise non-responsive to stimuli; increased amounts of vitamin A are known to counteract that non-responsiveness, and thereby facilitate the promotion ofwound healing. Similarly, increasing the level of vitamin E is believed to facilitate the promotion of wound healing.

The Knighton patent discloses the use of isolated multiple growth factors combined with a biologically compatible carrier substance, after sequestration (and removal) of all platelet membranes and plasma containing fibrin from the growth factor exudate prepared. Discarding such membranes essentially removes from the composition residual growth factors known to be concentrated in the membranes, and potential receptor sites for facilitating matrix formation. The method utilized in Knighton also requires a number of time consuming and labor intensive steps, including storage at -20° to -30° centigrade prior to use. The Knighton method also requires that wound treatments be repeated on a daily basis.

The Antanavich patent discloses a composition based primarily on plasma and, like Knighton, requires a biologically acceptable carrier for administering a plasma concentrate comprising platelets, fibrinogen and fibrinectin. The Antanavich composition is essentially a fibrin glue meant to have a high tensile strength (viscosity), sufficient to seal a wound.

To the extent that the Martin patent is relevant, Martin discloses a composition comprising a sunscreen agent, an anti-inflammatory agent, and a wound healing composition. (Martin, column 6 line 66 through column 7 line 2.) Said wound healing composition comprises the combination of pyruvate, an anti-oxidant (including vitamins A, C and E), and fatty acids required for repairing cellular membranes. (Martin, column 7 lines 2 through 8.) The utility and function of said vitamins to the Martin composition, intended for use in sunlight rather than shielded from sunlight, are distinctly different from the utility and function of the vitamins to the invention disclosed herein, as explained hereinbelow.

The chemical reactions and cascades that normally happen when thrombin is added to the concentrated platelets are indeed complex. They are discussed in the scientific article by Reeder, et al, in Proceedings of the American Academy of Cardiovascular Perfusion, Vol. 14, January 1993. Adding a preservative, or healing promotion materials that do not detract from, substantially interfere with, or even destroy these different reactions is the crux of the invention disclosed herein.

One object of the invention is to provide a wound treatment material that is capable of quick and convenient production in the presence of the patient, that facilitates the promotion of wound healing, and that facilitates the prevention of wound infection.

Another object is to provide a method of making a wound treatment material satisfying the objectives expressed.

### SUMMARY OF THE INVENTION

In most general terms, the invention described herein expands the uses for concentrated platelet materials, especially those in gel form, by improving the speed and convenience of making the composition, the invention described herein also improves the performance of the concentrated platelet composition, by making it more useable for applications over longer periods of time, and by enhancing the wound healing and infection fighting properties. For autologous platelet gel to be more useful, the gelatinous state must be capable of remaining stable for a reasonable period of time. One aspect of the present invention is to add a preservative to the platelet gel, such as ascorbic acid.

In a preferred embodiment of the invention there is provided a method of making a wound healant composition comprising providing a therapeutically effective amount of activated growth factors by activating a composition comprising concentrated platelets and including an effective gel viscosity preservation amount of ascorbic acid.

In a further preferred embodiment of the invention there is provided a method of making a wound healant, comprising the steps of mixing therapeutically effective amounts of concentrated platelets, ascorbic acid, at least one retinoid and at least one antibiotic which is bacteriocidal to at least Pseudomonas and Klebsella bacteria, and then admixing thrombin.

In a further preferred embodiment there is provided a wound healant composition comprising a therapeutically effective amount of activated growth factors that originate in concentrated platelets, an effective gel viscosity preservation amount of ascorbic acid, and an activating agonist selected from the group consisting of thrombin, collagen, serotonin, adenosine disphosphate (ADP) and acetylcholine (ACH) and combinations thereof, which agonist produces said growth factors from said concentrated platelets.

Another aspect of the present invention involves adding one or more antibiotic substance at one or more times during the processing period so that the resulting concentrated platelet composition contains either one or a variety of the antibiotics. The embodiments disclosed herein involves adding antibiotic compositions in a manner that does not detract from, substantially interfere with, or even destroy these different reactions, pH balances and potency.

Another embodiment of the present invention involves adding one or more vitamins known to promote wound healing, such as vitamin A and vitamin E.

The method of making the invention in gel form described herein includes mixing at least one of the described additives with the plasma-poor concentrated platelets, a sufficient time before the addition of calcified thrombin (or other preferably-calcified agonist) to allow the desired dispersion of such additive(s) in such composition before gelation prevents further dispersion.

### DETAILED DESCRIPTION OF THE INVENTION

The biological materials specified herein may originate from a patient to be treated, from a single third party, or from a plurality of third parties; moreover, said third parties may be of the same species as the patient, or of another species, so long as the wound treatment material derived from such biological materials is biocompatible with the patient.

It is also to be understood that the terminology used herein is not intended to be limiting, since the scope of the present invention will be limited only by the claims and equivalents thereof Also, as used herein, the singular forms include the plurals, and vice versa, unless the context indicates otherwise.

In most general terms, the invention includes a wound healant composition comprising activated growth factors and ascorbic acid. In the prevalent version of the invention, said growth factors are included within platelets. The body produces many substances generally known as growth factors, and the growth factors of the present invention are selected from the group consisting of platelet-derived growth factor (PDGF), platelet-derived angiogenesis factor (PDAF), vascular endotheial growth factor (VEGF), platelet-derived epidermal growth factor (PDEGF), platelet factor 4 (PF-4), transforming growth factor P (TGF-B), acidic fibroblast growth factor (FGF-A), basic fibroblast growth factor (FGF-B), transforming growth factor α (TGF-A), insulin-like growth factors 1 and 2 (IGF-1 and IGF-2), β thromboglobulin-related proteins (BTG), thrombospondin (TSP), fibronectin, von Wallinbrand's factor (vWF), fibropeptide A, fibrinogen, albumin, plasminogen activator inhibitor 1 (PAI-1), osteonectin, regulated upon activation normal T cell expressed and presumably secreted (RANTES), gro-α, vitronectin, fibrin D-dimer, factor V, antithrombin III, immunoglobulin-G (IgG), immunoglobulin-M (IgM), immunoglobulin-A (IgA), a2-macroglobulin, angiogenin, Fg-D, elastase, keratinocyte growth factor (KGF), epidermal growth factor (EGF), fibroblast growth factor (FGF), tumor necrosis factor (TNF), fibroblast growth factor (FGF) and interleukin-1 (IL-1), and combinations thereof. One of the important characteristics common to each substance, supporting the inclusion of each in this particular group, is that each such substance is known or believed to enhance cell or tissue growth. Moreover, said substances, or various combinations thereof, are known or believed to function together in an unexpected synergistic manner to promote wound healing.

The platelets are separated from the red blood cells and white blood cells of whole blood, primarily through differential centrifugation. However, the overall composition of the invention disclosed herein may contain incidental amounts of white blood cells, due to the fact that the platelets are rarely totally isolated from the other blood components. It is believed that the present invention contains only minimal or trace amounts of white blood cells; it is believed that the white blood cell count of the present invention typically will be below about 3 times 10⁷ cells/ml. The present invention does not remove water to achieve concentration of cells. The invention biomaterials is almost exclusively from platelets. The range of the mean platelet volume of the platelets being sequestered is in the range of about 6.6 to 8.4 femtoliters, with an average of about 7.7 femtoliters; this may indicate that the platelets being sequestered are relatively larger or younger than the overall population of platelets.

Activation of growth factors may occur in a variety of manners, by a variety of substances known as activators or agonists. In the invention described herein, said activation results from the inclusion of an activator or agonist selected from the group consisting of thrombin, collagen, serotonin, adenosine diphosphate (ADP) and acetylcholine (ACH), and combinations thereof In a particular version of the invention, said growth factors are included within concentrated platelets, and said activation results from the inclusion of thrombin. One of the important characteristics common to each substance, supporting the inclusion of each in this particular group, is that each such substance is known or believed to enhance cell or tissue growth. Moreover, said substances, or various combinations thereof, are known or believed to function together in an unexpected synergistic manner to promote wound healing.

The invention is not limited to autologous biological materials, such as where said concentrated platelets are obtained from the wounded's own biological material. The invention encompasses the use of biological materials obtained from one or more third parties, that need not be of the same species as the patient whose wound is being treated with the wound healant composition described herein unless bioincompatibility would result from the use of such third party biological materials.

In one general version of the invention, the wound healant composition includes concentrated platelets, thrombin and ascorbic acid. Ascorbic acid is known to have preservative properties, unless it is broken down such as occurs after exposure to sunlight or another source of ultraviolet (UV) light rays. However, most versions of the invention described herein are covered by bandages or otherwise shielded from UV rays almost immediately after application to the wound site.

Since the admixture of thrombin or other agonists will activate growth factors, the thrombin (or other agonists/activators) should usually be the last substance to be mixed immediately before it is desired that the gelatinous state be set up.

Another version of the invention includes the inclusion of at least one retinoid in the admixture, in addition to or in substitution of the ascorbic acid. Although the wound healant composition could include a combination of retinoids, one version of the invention merely includes vitamin A in addition to or in substitution of the ascorbic acid. Vitamin A has been known to counteract a side effect of some treatments using steroids, namely, the depressed reactivity of the body immune system to stimuli. Furthermore, vitamin A is known or believed to inhibit or decrease the bioactivity of manganese, magnesium and copper in the cellular and interstitial environment; said elements are known or believed to be active or instrumental in the laying down of keloids and scar tissue. (See, Effects of Pantothenic Acid and Ascorbic Acid Supplementation on Human Skin Wound Healing Process by Vaxman et al., Eur. Surg. Res. 1995, 28:4, 158-166.) The versions of the invention described herein containing vitamin A accordingly are believed to promote healing without as much scarring or keloid formation. In another version, said retinoid is vitamin E, known to facilitate healing. In any event, the vitamins disclosed herein (or combinations thereof) appear to enhance wound healing in an unexpectedly synergistic manner.

The utility and function of said vitamins are independent of any anti-oxidative properties. It should be recognized that none of said vitamins in this invention likely exhibits any anti-oxidative properties when applied topically and exposed to UV rays, as in Martin. UV rays rapidly break down such vitamins, or otherwise render them virtually impotent; moreover, any anti-oxidative properties are exhibited when the vitamin is absorbed internally, not via mere topical application.

Furthermore, the utility and function of said vitamins to the invention disclosed herein have other distinct differences from the utility and function of the vitamins to the Martin composition. For example, the ascorbic acid lowers the pH of the surrounding media, and thus makes it more difficult for saprophytic bacteria to grow in the wound bed.

Although the wound healant composition could include a combination of antibiotics, one version of the invention merely substitutes at least one antibiotic in addition to or in substitution of the ascorbic acid. Since many wound sites are either already infected with bacteria or are susceptible to such infection, it is desirable that a wound healant composition be capable of either killing bacteria or preventing the mobility or reproduction of bacteria. The invention described herein includes a wound healant composition comprising concentrated platelets, thrombin and at least one antibiotic. In particular, the invention includes a wound healant wherein said antibiotic is bacteriocidal to at least the *Pseudomonas* and *Klebsella* genera of bacteria, which are prevalent at wound sites and difficult to guard against. Alternatively, said antibiotic is selected from the group consisting of a neosporin, vancomycin and gentamycin, and combinations thereof One of the important characteristics common to each substance, supporting the inclusion of each in this particular group, is that each such substance is known to kill said bacteria.

As indicated above, the invention may include a wound healant composition comprising concentrated platelets, thrombin, ascorbic acid, at least one retinoid and at least one antibiotic bacteriocidal to the *Pseudomonas* and *Klebsella* genera of bacteria.

Aside from the wound healant substance to be applied to wound sites, the invention further includes a method of making a wound healant composition. One manner of making the plasma-poor concentrated platelets of the present invention is to collect about 450 ml of whole blood in anticoagulant (such as sodium citrate or any similar anticoagulant known in the field). That blood is then centrifuged at at least one speed in the range of between about 2,000 rpms and 3,000 rpms (preferably about 2,400 rpms) for a duration of between about 15 minutes and 25 minutes (preferably about 20 minutes) to separate out a band (or similar grouping) of: (a) plasma and most white blood cells; (b) platelets (and incidental white blood cells); and (c) red blood cells. The platelet portion may then be re-centrifuged to further separate out plasma (and incidental white blood cells); said re-centrifuging may be at at least one speed in the range of between about 4,000 rpms and 5,600 rpms (preferably about 4,800 rpms) for a duration of between about 5 minutes and 10 minutes (preferably about 7½ minutes).

The final yield is about 40 to 50 ml of plasma-poor concentrated platelets (in trace or incidental amounts of residual plasma and white blood cells). Both the plasma/leukocyte portion and the red blood cell portion may be reinfused back into the patient. The plasma-poor concentrated platelets may then be activated by a mixture of thrombin and (preferably) calcium. Preferably, a solution comprising 5,000 units ofthrombin per ml of calcium chloride solution will result; since blood anticoagulants typically tie up blood calcium to prevent the clotting cascade from occurring, calcified thrombin is used to re-supply the plasma-poor concentrated platelets with more calcium to facilitate the clotting cascade at the wound site. Depending upon the relative concentrations of the ingredients, the resulting mixture may be either a liquid, or it may set up as a hard material or (preferably) as a gel having a viscosity dependant upon the relative amounts of thrombin and platelets; the relative concentrations of calcified thrombin to platelets determines how quickly the composition sets up, and how hard it will eventually be. Some mixtures will yield a composition that will set up in a gel in several seconds, whereas some mixtures will yield a composition that takes several minutes to set up in a gel.

Regardless of the amount of set up time, the present invention includes a preservative that allows the gel to retain its viscosity for a longer duration. For example, ascorbic acid is believed to preserve the longevity of the gel viscosity. Another method ofmaking the wound healing composition includes the steps of mixing activated growth factors with ascorbic acid. Said activated growth factors may be obtained in a variety of ways, such as by the steps of sequestering concentrated platelets from blood and mixing thrombin with said platelets. Said ascorbic acid should be in sufficient amount to enhance the preservation of the gelatinous state of the final wound healing composition, and said thrombin should be in sufficient amount to facilitate formation of the coagulum (gel) having the desired level of viscosity while sufficiently activating growth factors present in the composition and the wound.

In one preferred version of the composition, about 1 ml of ascorbic acid is mixed with about 8 ml of concentrated platelets, then about 1 ml of calcified thrombin is mixed into that 9 ml admixture. However, other ratios of concentrated platelets: ascorbic acid:thrombin may be useful, depending upon the desired amount of healing agents, gelation time, gel viscosity and longevity.

Another method of making the composition allows the extraction of blood, sequestering of plasma-poor concentrated platelets, mixing of additives and return of unused blood components to the patient, all in about 20 to 30 minutes and by making only one puncture in the patient. Approximately 125 to 250 ml of blood is extracted from a patient, with the blood drawing apparatus optionally remaining in place connected to the patient (for later use in returning unused blood components to the patient). That blood is transferred to a Lathum bowl and, using a centrifuge such as is manufactured by Haemonetics, Inc., centrifuged at about 4,800 rpms until a band (or similar grouping) of plasma forms at the upper periphery (about 5 to 15 minutes), and a band (or similar grouping) of red blood cells forms at the bottom of the bowl; the center is comprised of plasma-poor concentrated platelets. The plasma band is removed for return to the patient, and the remaining blood components are again centrifuged at that speed (and sufficient duration), further removing plasma and white blood cells from the plasma-poor concentrated platelets. The plasma-poor concentrated platelets are then removed for mixing with the other additives described herein (thrombin, ascorbic acid and/or retanoids).

The method of making a wound healant may further include, prior to or contemporaneous with mixing said thrombin, mixing at least one of the aforementioned retinoids in sufficient amount(s) to further enhance wound healing. In one version, 1 ml of aqueous vitamin A and vitamin E solution is added to 8 ml of concentrated platelets and 1 ml of ascorbic acid, before mixing in 1 ml of thrombin.

Alternatively, said method may include, prior to or contemporaneous with mixing said thrombin, mixing at least one of the aforementioned antibiotics in sufficient amount(s) to reduce infection by bacteria.

The wound healant composition of the invention may be used for treating a wound, by applying a sufficient amount of said wound healant composition to enhance healing of the wound.

Once applied to a wound, the composition may remain on the wound for as long as 5 days, and perhaps longer depending upon the circumstances such as the location of the wound and other wound characteristics. Although the composition described herein is especially useful for the treatment of chronic wounds, it may also be useful in the treatment of acute wounds.

### Example 1

Case study: Patient P is a 57 year old white male truck driver with a right heel diabetic ulcer of 11 month's duration. His treatment regimen has consisted of rest, off-loading and daily wound cleansing with soap and water followed by application of gauze dressing. Carrasyn gel was ordered for a brief period, without improvement. Upon referral to an outpatient physical therapy department for wound treatment, P's current therapy consists of weekly sharp debridement, wet saline gauze and total contact cast P has history of hypertension, which is controlled at present time. He has a 15-year history of diabetes mellitus with neuropathy, which is controlled with oral hypoglycemic elements.

P began treatment with the invention disclosed herein. After his wound was sharply debrided, the gel coagulum was applied and the wound was covered with a wet saline dressing. A total contact cast was then applied and left intact for one week. At the conclusion of week 1 the cast was removed, the wound site cleansed and recovered with wet dressing; the limb was re-cast for week 2. After the conclusion of week 2, the same procedure was followed, except that the gel coagulum was again applied to the wound site before covering with wet dressing. After the conclusion of week 3 the same procedure as for week 2 was followed. This regimen continued for a total of 36 days. Table 1 below contains the data reflecting the reduction in wound site volume and surface area during weeks 1 through 4.

**Table 1**

| **Week #** | **Volume (mm³)** | **Area (mm²**) |
|---|---|---|
| **0** | 3121 | 674 |
| **1** | 1561 | 562 |
| **2** | 279 | 301 |
| **3** | 26 | 282 |
| **4** | 15 | 159 |

### Example 2

| **Pt #** | **Volume (mm³)** | | **Area (mm²)** | |
|---|---|---|---|---|
| | ***Start*** | ***End*** | ***Start*** | ***End*** |
| **1** | 3121 mm³ | 15 mm³ | 674 mm² | 159 mm² |
| **2** | 358 mm³ | 0.0 mm³ | 65 mm² | 4 mm² |
| **3** | 293 mm³ | 3.0 mm³ | 63 mm² | 3 mm² |
| **4** | 192 mm³ | 18 mm³ | 104 mm² | 39 mm² |
| **5** | 336 mm³ | 0.0 mm³ | 181 mm² | 0.0 mm² |

The five patients that entered into this study were referrals by their physicians. The patients were then screened using the exclusion, inclusion criteria. The patients selected for study had an ulcer of the lower extremity that had not healed after four to six months of treatment either with traditional wound care alone, or with traditional care plus *Regranex*¹.
¹ A single growth factor product of Ortho-McNeal marketed by Johnson & Johnson.

All five of the study patients had a platelet count of 100,000 cells/mm³ or greater, and had a hemoglobin >10 g and a HCT of 30% or greater. The patients were evaluated for infection in the wound, and for osteomyelitis. None of the patient studied showed signs of infection, or bone involvement.

Aggressive debridement to essentially change the chronic wound to an acute one was used. The ulcer and surrounding callus were completely excised down to normal uninvolved tissue. All subjects were treated as outpatients. All patients agreed to be totally non-weight-bearing. With the exception of one, patients were supplied with a half-shoe that transferred weight to the unaffected area of the foot. The one patient not fitted with the half-shoe was fitted with a full cast of the lower leg. Direct questioning of patients and family assessed the compliance issue. Only one patient proved to be non-compliant. Patient 4's blood sugar exceeded 400mg/dl and she became disoriented and walked on her foot at post treatment day 2. This resulted in a re-treatment of patient 4². Table 2 shows the wound size and volume at the commencement and conclusion of treatment with the coagulum disclosed herein. Closure, on the average, took less than four weeks. The average patient wound was brought to 99% closure in 25 days.
² Pt#4 NG was non-compliant through out this study, missing three clinic visits.

## Claims

1. A method of making a wound healant composition comprising providing a therapeutically effective amount of activated growth factors by activating a composition comprising concentrated platelets and including an effective gel viscosity preservation amount of ascorbic acid.

2. A method according two claim 1, wherein the activation results from the inclusion of an agonist selected from the group consisting of thrombin, collagen, serotonin, adenosine diphosphate (ADP) and acetylcholine (ACH), and combinations thereof.

3. A method according to claim 1, wherein said activation occurs by admixing the composition with thrombin.

4. A method according to claim 3 wherein said concentrated platelets are separated from other blood components by centrifuging blood at at least one speed in the range of between 2,000 rpms and 3,000 rpms for between 15 minutes and 25 minutes, then further concentrated by centrifuging same at at least one speed in the range of between 4,000 rpms and 5,600 rpms for 5 to 10 minutes, and is thereafter mixed with ascorbic acid and then with thrombin in sufficient amounts to result in gelation having a desired level of viscosity and longevity, and having therapeutically effective amounts of healant.

5. A method according to claim 4, wherein:
for the first separating said platelets, said centrifuge speed is about 2,400 rpms and said centrifugation duration is about 20 minutes;
for further concentrating said platelets, said centrifuge speed is about 4,800 rpms and said centrifugation duration is about 7½ minutes; and
1 ml of said ascorbic acid is mixed in 8 ml of said concentrated platelets.

6. A method according to any one oc claims 3 to 5 wherein said thrombin amount is about 5,000 units in about 1.0 ml of aqueous calcium chloride solution.

7. A method according to any one of claims 3 to 6, said method further comprising, admixing at least one retinoid in sufficient amount(s) to further enhance wound healing prior to mixing said thrombin.

8. A method according to claim 7 wherein said retinoid is vitamin A in sufficient amount to reduce any non-responsiveness of the wounded's immune system to stimuli, and vitamin E in sufficient amount to further enhance wound healing.

9. A method according to claim 8 wherein about 1 ml of vitamin A and vitamin E is mixed with about 8 ml of said concentrated platelets and about 1 ml of said ascorbic acid, then mixed with about 5,000 units of thrombin in about 1.0 ml of aqueous calcium chloride solution.

10. A method according to any one of claims 3 to 9 comprising admixing at least one antibiotic in sufficient amount(s) to reduce infection by bacteria prior to mixing said thrombin.

11. A method according to claim 10 wherein said antibiotic is at least bacteriocidal to Pseudomonas and Klebsella bacteria.

12. A method according to claim 10 wherein said antibiotic is selected from the group consisting of neosporin, vancomycin and gentamycin, and combinations thereof.

13. A method of making a wound healant comprising the steps of:
mixing therapeutically effective amounts of concentrated platelets, ascorbic acid, at least one retinoid and at least one antibiotic which is bacteriocidal to at least Pseudomonas and Klebsella bacteria, and
then admixing thrombin.

14. A wound healant composition comprising:
a therapeutically effective amount of activated growth factors that originate in concentrated platelets,
an effective gel viscosity preservation amount of ascorbic acid, and
an activating agonist selected from the group consisting of thrombin, collagen, serotonin, adenosine diphosphate (ADP) and acetylcholine (ACH) and combinations thereof, which agonist produces said growth factors from said concentrated platelets.

15. A wound healant composition according to claim 14, wherein said agonist is thrombin.

16. A wound healant composition according to claim 14 or 15 wherein said platelet concentrate is obtained from the wounded's own biological material which has a white blood cell count of below 3 times 10⁷ cells/ml.

17. A wound healant composition according to claim 14 which additionally comprises at least one retinoid, at least one antibiotic bacteriocidal to at least Pseudomonas and Klebsella, and thrombin.

18. The wound healant composition of claim 14 or 17 comprising about 8 ml of concentrated platelets, about 1 ml of ascorbic acid and about 1 ml of thrombin.

19. The wound healant composition of claim 18, further comprising about 1 ml of vitamin A and vitamin E.

## Patentansprüche

1. Verfahren zur Herstellung einer Wundheilmittelzusammensetzung, das die Bereitstellung einer therapeutisch wirksamen Menge aktivierter Wachstumsfaktoren durch Aktivieren einer Zusammensetzung, die konzentrierte Blutplättchen umfasst, und die Zugabe einer wirksamen Gelviskositäterhaltungsmenge von Ascorbinsäure beinhaltet.

2. Verfahren nach Anspruch 1, wobei die Aktivierung von der Zugabe eines Agonisten resultiert, der ausgewählt ist aus der Gruppe bestehend aus Thrombin, Kollagen, Serotonin, Adenosindiphosphat (ADP) und Acetylcholin (ACH) und Kombinationen davon.

3. Verfahren nach Anspruch 1, wobei die genannte Aktivierung durch Mischen der Zusammensetzung mit Thrombin erfolgt.

4. Verfahren nach Anspruch 3, wobei die genannten konzentrierten Blutplättchen von anderen Blutkomponenten abgeschieden werden, indem Blut 15 bis 25 Minuten lang mit wenigstens einer Drehzahl im Bereich zwischen 2000 und 3000 rpm zentrifugiert, dann durch Zentrifugieren desselben 5 bis 10 Minuten lang mit wenigstens einer Drehzahl im Bereich zwischen 4000 und 5600 rpm weiter konzentriert und dann mit Ascorbinsäure und danach mit Thrombin in ausreichenden Mengen gemischt wird, so dass eine Gelierung mit einem/r gewünschten Viskositätsniveau und Langlebigkeit erzielt wird, und mit therapeutisch wirksamen Mengen des Heilmittels.

5. Verfahren nach Anspruch 4, wobei:
für das erste Abscheiden der genannten Blutplättchen die genannte Zentrifugaldrehzahl etwa 2400 rpm und die genannte Zentrifugationsdauer etwa 20 Minuten beträgt;
für das weitere Konzentrieren der genannten Blutplättchen die genannte Zentrifugaldrehzahl etwa 4800 rpm und die genannte Zentrifugationsdauer etwa 7½ Minuten betragen; und
1 ml der genannten Ascorbinsäure mit 8 ml der genannten konzentrierten Blutplättchen gemischt wird.

6. Verfahren nach einem der Ansprüche 3 bis 5, wobei die genannte Thrombinmenge etwa 5000 Einheiten in etwa 1,0 ml wässriger Calciumchloridlösung beträgt.

7. Verfahren nach einem der Ansprüche 3 bis 6, wobei das genannte Verfahren ferner das Mischen von wenigstens einem Retinoid in ausreichender/n Menge(n) beinhaltet, um die Wundheilung vor dem Mischen mit dem genannten Thrombin zu verbessern.

8. Verfahren nach Anspruch 7, wobei das genannte Retinoid Vitamin A in einer ausreichenden Menge ist, um ein Nichtansprechen des Immunsystems des Verwundeten auf Reize zu reduzieren, und Vitamin E in ausreichender Menge ist, um die Wundheilung noch weiter zu verbessern.

9. Verfahren nach Anspruch 8, wobei etwa 1 ml Vitamin A und Vitamin E mit etwa 8 ml der genannten konzentrierten Blutplättchen und etwa 1 ml der genannten Ascorbinsäure und dann mit etwa 5000 Einheiten Thrombin in etwa 1,0 ml wässriger Calciumchloridlösung gemischt werden.

10. Verfahren nach einem der Ansprüche 3 bis 9, das das Mischen mit wenigstens einem Antibiotikum in ausreichender/n Menge(n) umfasst, um Bakterieninfektionen vor dem Mischen des genannten Thrombin zu reduzieren.

11. Verfahren nach Anspruch 10, bei dem das genannte Antibiotikum wenigstens für Pseudomonas- und Klebsella-Bakterien bakteriozidal ist.

12. Verfahren nach Anspruch 10, wobei das genannte Antibiotikum ausgewählt ist aus der Gruppe bestehend aus Neosporin, Vancomycin und Gentamycin und Kombinationen davon.

13. Verfahren zur Herstellung eines Wundheilmittels, das die folgenden Schritte beinhaltet:
Mischen therapeutisch wirksamer Mengen von konzentrierten Blutplättchen, Ascorbinsäure, wenigstens einem Retinoid und wenigstens einem Antibiotikum, das für wenigstens Pseudomonas- und Klebsella-Bakterien bakteriozidal ist, und
dann Beimischen von Thrombin.

14. Wundheilmittelzusammensetzung, die Folgendes umfasst:
eine therapeutisch wirksame Menge aktivierter Wachstumsfaktoren, die von konzentrierten Blutplättchen stammen,
eine effektive Gelviskositäterhaltungsmenge von Ascorbinsäure, und
einen Aktivierungsagonisten, ausgewählt aus der Gruppe bestehend aus Thrombin, Kollagen, Serotonin, Adenosindiphosphat (ADP) und Acetylcholin (ACH) und Kombinationen davon, wobei dieser Agonist die genannten Wachstumsfaktoren von den genannten konzentrierten Blutplättchen produziert.

15. Wundheilmittelzusammensetzung nach Anspruch 14, wobei der genannte Agonist Thrombin ist.

16. Wundheilmittelzusammensetzung nach Anspruch 14 oder 15, wobei das genannte Blutplättchenkonzentrat vom eigenen biologischen Material des Verwundeten genommen wurde, das eine Zahl von weißen Blutkörperchen von weniger als 3 x 10⁷ Zellen/ml hat.

17. Wundheilmittelzusammensetzung nach Anspruch 14, die zusätzlich wenigstens ein Retinoid, wenigstens ein Antibiotikum, das gegenüber wenigstens Pseudomonas und Klebsella bakteriozidal ist, und Thrombin umfasst.

18. Wundheilmittelzusammensetzung nach Anspruch 14 oder 17, die etwa 8 ml konzentrierte Blutplättchen, etwa 1 ml Ascorbinsäure und etwa 1 ml Thrombin umfasst.

19. Wundheilmittelzusammensetzung nach Anspruch 18, die ferner 1 ml Vitamin A und Vitamin E umfasst.

## Revendications

1. Procédé de fabrication d'une composition cicatrisante pour plaies comprenant fournir une quantité thérapeutiquement efficace de facteurs de croissance activés en activant une composition comprenant des plaquettes concentrées et incluant de l'acide ascorbique en une quantité efficace pour conserver la viscosité d'un gel.

2. Procédé selon la revendication 1, dans lequel l'activation résulte de l'inclusion d'un agoniste sélectionné parmi le groupe consistant en thrombine, collagène, sérotonine, adénosine diphosphate (ADP) et acétylcholine (ACH) et des combinaisons de ceux-ci.

3. Procédé selon la revendication 1, dans lequel ladite activation survient en ajoutant et en mélangeant la composition avec de la thrombine.

4. Procédé selon la revendication 3, dans lequel lesdites plaquettes concentrées sont séparées d'autres composants du sang en centrifugeant le sang à au moins une vitesse dans la plage d'entre 2.000 rpm et 3.000 rpm pendant entre 15 minutes et 25 minutes, puis concentrées encore en centrifugeant les mêmes à au moins une vitesse dans la plage d'entre 4.000 rpm et 5.600 rpm pendant 5 à 10 minutes, et elles sont ensuite mélangées avec de l'acide ascorbique et puis avec de la thrombine en des quantités suffisantes pour résulter en ce que la gélification ait un niveau désiré de viscosité et de longévité, et ayant des quantités thérapeutiquement efficaces de cicatrisant.

5. Procédé selon la revendication 4, dans lequel :
pour séparer premièrement les plaquettes, ladite vitesse de centrifugation est d'environ 2.400 rpm et ladite durée de centrifugation est d'environ 20 minutes;
pour concentrer encore lesdites plaquettes, ladite vitesse de centrifugation est d'environ 4.800 rpm et ladite durée de centrifugation est d'environ 7 ½ minutes; et
1 ml dudit acide ascorbique est mélangé dans 8 ml desdites plaquettes concentrées.

6. Procédé selon l'une quelconque des revendications 3 à 5, dans lequel ladite quantité de thrombine est d'environ 5.000 unités dans environ 1,0 ml de solution aqueuse de chlorure de calcium.

7. Procédé selon l'une quelconque des revendications 3 à 6, ledit procédé comprenant en outre ajouter et mélanger au moins un rétinoïde en une/des quantité(s) suffisante(s) pour favoriser encore la cicatrisation des plaies avant de mélanger ladite thrombine.

8. Procédé selon la revendication 7, dans lequel ledit rétinoïde est de la vitamine A en une quantité suffisante pour réduire toute non réponse du système immun du blessé à des stimulus, et de la vitamine E en une quantité suffisante pour favoriser encore la cicatrisation des plaies.

9. Procédé selon la revendication 8, dans lequel environ 1 ml de vitamine A et de vitamine E est mélangé avec environ 8 ml desdites plaquettes concentrées et environ 1 ml dudit acide ascorbique, puis mélangé avec environ 5.000 unités de thrombine dans environ 1,0 ml de solution aqueuse de chlorure de calcium.

10. Procédé selon l'une quelconque des revendications 3 à 9, comprenant ajouter et mélanger au moins un antibiotique en une/des quantité(s) suffisante(s) pour réduire l'infection par des bactéries avant de mélanger ladite thrombine.

11. Procédé selon la revendication 10, dans lequel ledit antibiotique est au moins bactéricide pour les bactéries *Pseudomonas* et *Klebsiella*.

12. Procédé selon la revendication 10, dans lequel ledit antibiotique est sélectionné parmi le groupe consistant en néosporine, vancomycine et gentamycine et des combinaisons de celles-ci.

13. Procédé de fabrication d'un cicatrisant pour plaies comprenant les étapes consistant à:
mélanger des quantités thérapeutiquement efficaces de plaquettes concentrées, d'acide ascorbique, d'au moins un rétinoïde et d'au moins un antibiotique qui est bactéricide pour au moins *Pseudomonas* et *Klebsiella*, et
puis ajouter et mélanger de la thrombine.

14. Composition cicatrisante pour plaies comprenant :
une quantité thérapeutiquement efficace de facteurs de croissance activés qui proviennent de plaquettes concentrées,
de l'acide ascorbique en une quantité efficace pour conserver la viscosité d'un gel, et
un agoniste activant sélectionné parmi le groupe consistant en thrombine, collagène, sérotonine, adénosine diphosphate (ADP) et acétylcholine (ACH) et des combinaisons de ceux-ci, lequel agoniste produit lesdits facteurs de croissance desdites plaquettes concentrées.

15. Composition cicatrisante pour plaies selon la revendication 14, dans laquelle ledit agoniste est de la thrombine.

16. Composition cicatrisante pour plaies selon la revendication 14 ou 15, dans laquelle ledit concentré de plaquettes est obtenu du propre matériel biologique du blessé qui a une numération de globules blancs de moins de 3 x 10⁷ globules/ml.

17. Composition cicatrisante pour plaies selon la revendication 14, qui comprend en plus au moins un rétinoïde, au moins un antibiotique bactéricide pour au moins *Pseudomonas* et *Klebsiella*, et de la thrombine.

18. La composition cicatrisante pour plaies de la revendication 14 ou 17, comprenant environ 8 ml de plaquettes concentrées, environ 1 ml d'acide ascorbique et environ 1 ml de thrombine.

19. La composition cicatrisante pour plaies de la revendication 18, comprenant en outre environ 1 ml de vitamine A et de vitamine E.
